# EUROPÄISCHE PATENTSCHRIFT

(11) **EP 1 702 644 B1**
(45) Veröffentlichungstag und Bekanntmachung des Hinweises auf die Patenterteilung: **20.10.2010**
(21) Anmeldenummer: 06004187.8
(22) Anmeldetag: 02.03.2006
(51) Int. Cl.: A61N 1/05

(54) **Stimulationselektrode mit poröser Beschichtung**
Stimulation electrode with a porous coating
Electrode de stimulation munie d'un revêtement poreux

(30) Priorität: 18.03.2005 DE 102005013066
(43) Veröffentlichungstag der Anmeldung: 20.09.2006
(73) Patentinhaber: W.C. Heraeus GmbH, 63450 Hanau (DE)
(72) Erfinder: Krüger, Frank, Dr., 63486 Bruchköbel (DE); Keitel, Oliver, 63741 Aschaffenburg (DE); Specht, Heiko, 63739 Aschaffenburg (DE); Wachter, Hans-Jürgen, Dr., 63322 Rödermark (DE)
(74) Vertreter: Kühn, Hans-Christian

(56) Entgegenhaltungen:
- EP-A- 0 054 781
- DE-A1- 4 207 368
- US-A- 4 502 492
- US-A- 6 023 638
- US-A1- 2005 059 862

## Beschreibung

Die vorliegende Erfindung betrifft eine Elektrode für medizinische Anwendungen, insbesondere zur Stimulation von Gewebe und ein geeignetes Herstellungsverfahren. Solche Elektroden werden in der Regel mit einer Beschichtung versehen.

Die EP 117 972 A, die EP 116 280 A sowie die EP 115 778 A offenbaren Elektroden für medizinische Anwendungen, die mit porösen Schichten aus Titannitrid versehen sind.

Gemäß EP 0 054 781 wird eine Herzschrittmacherelektrode mit geringer Stimulationsenergie bereitgestellt, die eine Beschichtung aus Platin, Iridium oder einer bis zu 20 Gew-% Iridium enthaltenden Platin-Iridium-Legierung aufweist. Die Beschichtung erfolgt in den Beispielen galvanisch.

Nach J. Riedmüller et al. Proceedings of the 14th annual conferece of the IEEE/EMBS, Orlando (1992) 2364-2365 wird die Oberfläche der Beschichtung vergrößert, um eine höhere Kapazität zu erlangen und damit die Impedanz des Übergangs von der Elektrode auf die Zelle zu verringern. Dabei kann die Fläche vergrößert werden, indem entweder Platin-Iridium-Spheroide aufgetragen und gesintert werden, oder eine glatte Fläche mit Platin-Black beschichtet wird. Als weitere Alternative lässt sich Iridium mittels Sputtertechnik auftragen.

Aus DE 42 07 368 ist eine Herzschrittmacherelektrode mit einer fraktalen gesputterten Iridium und Ir/Pt-Legierung Beschichtung bekannt. Iridium oxidiert bei der gebräuchlichen Anwendung zu IrO₂. Dies stellt insbesondere bei neurologischen Anwendungen, bei denen eine Anregung mit deutlich höherer Frequenz als bei der kardiologischen Stimulation stattfindet, ein erhebliches Problem dar, da durch die Umwandlung des Ir zu IrO₂ eine Degradation und Ablösung der Schicht im Körper erfolgen kann.

Weiterhin ist gemäß US 4,502,492 eine Platin-Black beschichtete Elektrode bekannt. Platin ist als Edelmetall zum einen wesentlich teurer als Iridium. Weiterhin ist aber auch die katalytische Wirkung des Platins weitreichender, so dass hier ggf. Nebeneffekte bei dem Kontakt mit Gewebe auftreten können.

Es ist die Aufgabe der vorliegenden Erfindung, unter minimiertem Einsatz von Edelmetall eine Beschichtung für Stimulationselektroden zur Erhöhung der elektrochemischen Kapazität bei gleichzeitiger Minimierung der katalytischen Aktivität bereitzustellen, wobei die Impedanz während der Stimulation auch unter anodischer Belastung stabil bleiben soll.

Die Lösung der Aufgabe erfolgt mit einem Sputterverfahren, bei dem die Beschichtung durch simultane Abscheidung von mindestens zwei Sputtertargets, davon mindestens einem Ir- und mindestens einem Pt-Target erfolgt und somit eine definierte Legierung einstellbar ist. Dabei kann die Stöchiometrie des Ptlr in der Beschichtung mit zunehmender Schichtdicke variiert werden. Somit wird ein Sputterverfahren bereitgestellt, bei dem eine einheitliche oder einen Gradienten aufweisende Legierung durch gleichzeitige Verwendung von Ir und Pt Targets in einem Sputterprozess auf einem zu besputternden Objekt erzeugbar ist.

Dieses Verfahren ermöglicht insbesondere die Herstellung einer einphasigen Platin-Iridium-Legierung, bei der das Iridium vollständig im Platin gelöst ist.

Auf diese Weise kann eine Stimulationselektrode hergestellt werden, die die gestellte Aufgabe mit einer Beschichtung aus einer Platin-Iridium-Legierung löst. Dabei ist die Legierungszusammensetzung frei wählbar und nicht durch die Zusammensetzung des Sputtertargets vorgegeben.

Der Platin-Anteil kann gering sein, um die katalytische Aktivität sowie die Kosten zu minimieren, wogegen Iridium als wesentlicher Bestandteil enthalten ist. Solche Elektroden mit geringem Platingehalt weisen eine hohe elektrochemische Kapazität auf, wie beispielsweise mehr als 5 mF/cm² in physiologischer Kochsalzlösung bei 37°C und einer Messfrequenz von 100 mHz.

Das erfindungsgemäße simultane Sputterverfahren mit zwei Targets ermöglicht nunmehr einen rationellen Legierungsauftrag mit individuell einstellbarer Legierungszusammensetzung auf dem einzelnen Objekt. Somit ist eine Serienherstellung mit unterschiedlich definierten Legierungen als Beschichtung ermöglicht. Als besonders geeignet haben sich dabei die Legierungen mit 5 bis 60 % Platin erwiesen. Besonders bevorzugt ist ein Pt-Gehalt von 10 bis 40 %.

Eine Lösung der vorliegenden Erfindung ist eine Elektrode für medizinische Anwendungen, die teilweise mit eienr porösen Platin-Iridium-Schicht beschichtet ist, wobei die Beschichtung mehr als 20 Gew-% Iridium, vorzugsweise mehr als 40 Gew-% Iridium, insbesondere mehr als 60 Gew-% Iridium und ganz besonders bevorzugt mehr als 80 Gew-% Iridium aufweist und mindestens 100 ppm Platin enthält und der beschichtete Bereich in physiologischer Kochsalzlösung bei 37°C und einer Messfrequenz von 100 mHz eine elektrochemische Kapazität über 5 mF/cm² aufweist.

In weiteren bevorzugten Ausführungen, die miteinander oder in Kombination anwendbar sind
■ ist das Platin vollständig im Iridium der Platin-Iridium-Beschichtung gelöst;
■ weist eine poröse Platin-Iridium-Beschichtung einen Gradienten der Stöchiometrie auf, insbesondere einen Gradienten, bei dem mindestens ein Legierungsbestandteil über die Schichtdicke um mindestens 10 Gew-% variiert.

Derartige Elektroden sind als Herzschrittmacherelektroden, Neurostimulationselektroden oder Muskelstimulationselektroden geeignet. Erfindungsgemäß lassen sich diese Elektroden in Serie individuell für den Einzelfall zugeschnitten, herstellen.

Nach Riedmüller et al. wird eine Erhöhung der elektrochemischen Kapazität erreicht, indem der Elektrodenkörper mit Ptlr-Kugeln beschichtet wird. Eine Herstellung von Ptlr-Kugeln ist jedoch aufgrund der mechanischen Eigenschaften von Ptlr-Legierungen nur bis zu einem Ir-Gehalt von 30 M.-% möglich. Um den Einsatz des teureren Platins weiter zu minimieren, sollte der Ir-Anteil größer 30 % betragen, was durch die Beschichtung beliebiger Zusammensetzungen aufgrund der simultanen Abscheidung von mindestens zwei Targets ermöglicht wird.

Erfindungsgemäß wird das Edelmetall aber besonders effizient genutzt, weil einerseits die Masse an Edelmetall besonders gering gehalten werden kann, dabei aber elektrochemische Kapazität maximiert wird und somit die Phasengrenzimpedanz zwischen Elektrode und Gewebe minimiert wird (Tabelle 1).

**Tabelle 1: Elektrochemische Kapazität bei f=1 Hz für verschiedene eingesetzte Edelmetallgewichte**

| | **Beschichtungsmasse** | **Kapazität bei f = 1 Hz** |
|---|---|---|
| Unbeschichtete Ptlr | - | 0,07 mF/cm² |
| Ptlr-Beschichtung nach J. Riedmüller et al. | ca. 10 mg | 2 mF/cm² |
| erfindungsgemäße Ptlr-Schicht | kleiner 0,5 mg | 19 mF/cm² |

Durch das erfindungsgemäße Verfahren der simultanen Abscheidung von mindestens zwei Targets, ist es weiterhin möglich, Ir-Schichten gezielt mit Pt zu dotieren, wobei die Pt-Dotierung mindestens 100 pm beträgt. Dadurch kann die im Einsatz als Stimulationselektrode erfolgende Oxidation des Iridiums zu IrO₂ vermindert werden. Eine Gefahr des Degradation und ggf. Ablösung der porösen Ptlr-Schicht besteht nicht.

Stimulationselektroden der vorliegenden Erfindung sind besonders für die Anwendung als Neurostirnulationselektrode geeignet, da hier mit einer gegenüber der kardialen Stimulation erhöhten Frequenz stimuliert wird und die Gefahr der Umwandlung des Ir zu IrO₂ besonders hoch ist.

Weitere geeignete Anwendungen sind die Verwendung der Elektrode als Herzschrittmacherelektrode oder als periphere Muskelstimulationselektrode.

Bevorzugtes Grundmaterial der Elektrode ist Titan, Tantal, Platin, Platin-Iridium oder Kobalt-Chrom-Legierung. Der bevorzugte Iridium-Gehalt der Platin-Iridium-Beschichtung liegt über 60 Gew-%, insbesondere über 80 Gew-%.

Im folgenden wird die vorliegende Erfindung anhand von Beispielen mit Bezug auf die Figuren verdeutlicht.

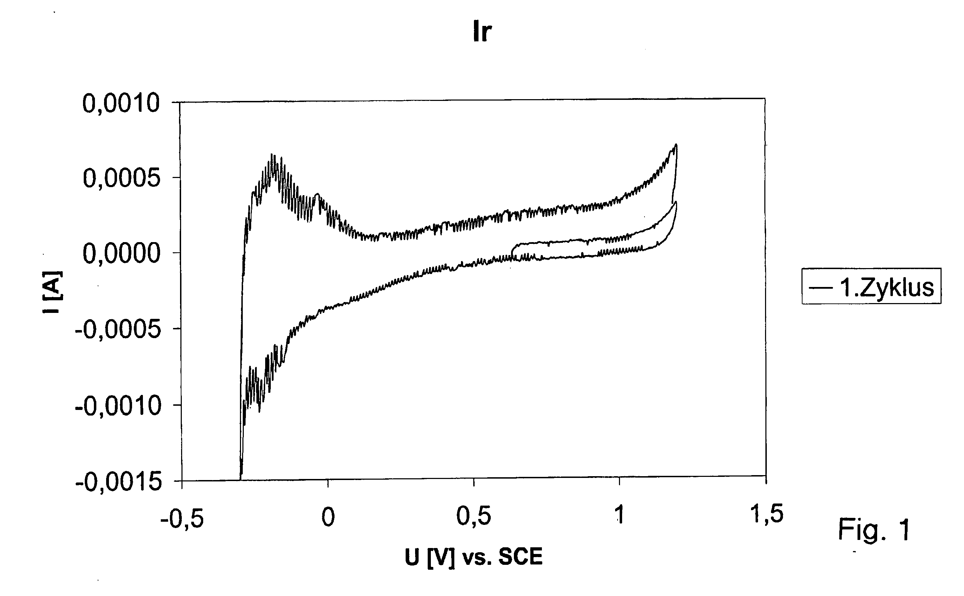

Figur 1 zeigt ein Zyklovoltammogramm (CV) des ersten Zyklus einer Iridiumelektrode gemessen gegen eine Standardkalomelelektrode (SCE);

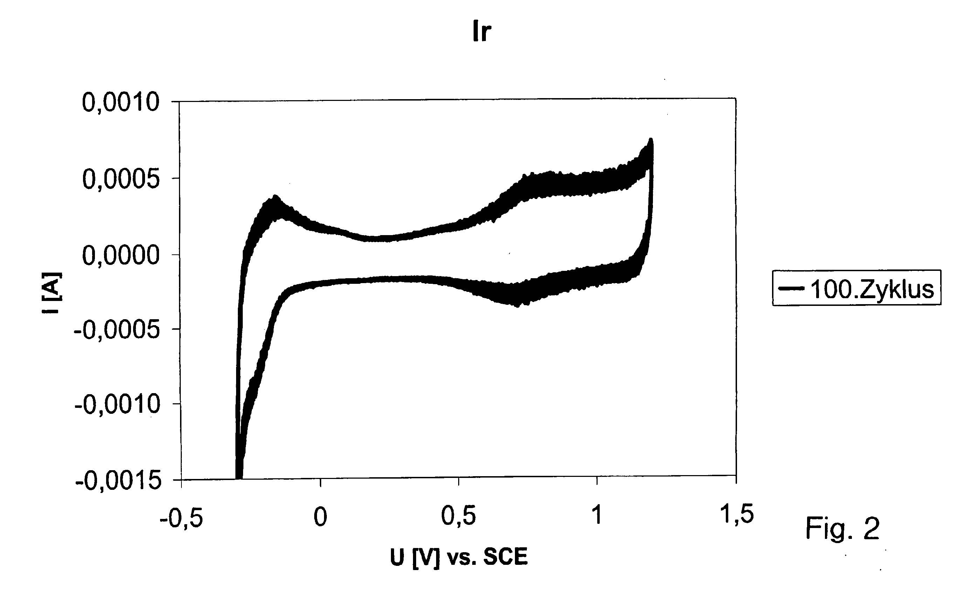

Figur 2 zeigt ein CV des 100. Zyklus einer Iridiumelektrode gemessen gegen eine Standardkalomelelektrode (SCE);

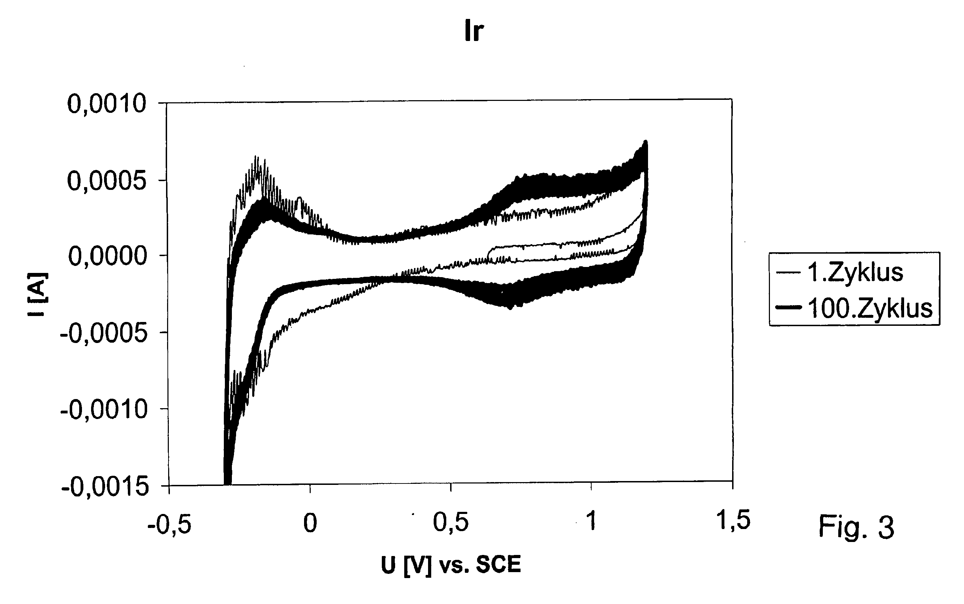

Figur 3 ist die Übereinanderlagerung von Figur 1 und 2;
Figur 4 zeigt ein CV des 1. Zyklus einer mit 5 % Platin dotierten Iridiumelektrode gemessen gegen eine Standardkalomelelektrode (SCE);
Figur 5 zeigt ein CV des 100. Zyklus einer mit 5 % Platin dotierten Iridiumelektrode gemessen gegen eine Standardkalomelelektrode (SCE);
Figur 6 zeigt die Übereinanderlagerung von Figuren 4 und 5;
Figur 7 zeigt ein CV des 1. Zyklus einer mit 10 % Platin dotierten Iridiumelektrode gemessen gegen eine Standardkalomelelektrode (SCE);
Figur 8 zeigt ein CV des 100. Zyklus einer mit 10 % Platin dotierten Iridiumelektrode gemessen gegen eine Standardkalomelelektrode (SCE);
Figur 9 zeigt eine Übereinanderlagerung der Figuren 7 und 8;
Figur 10 zeigt ein Pulverdiffraktogramm einer auf Glas gesputterten Iridium-Platin-Legierung. Hierbei ist zu erkennen, dass die Beschichtung aus einer einphasigen Legierung besteht;
Figur 11 zeigt die Impedanz einer Stimulationselektrode in Abhängigkeit von der Frequenz;
Figur 12 zeigt die Kapazitätszunahme durch Beschichtung mit Spheroiden und die weitere Kapazitätssteigerung mit einer erfindungsgemäß aufgetragenen Iridium-Platin-Legierung im Bereich von 70:30 bis 30:70.

Die Zyklovoltammogramme (CV) der mit Iridium beschichteten Herzschrittmacher (HSM)-Elektroden wurden analog zu Electroanalytical Chemistry and Interfacial Electrochemistry, 55 (1974) 375-381 © Elsevier Sequoia S.A., Lausanne in 1 M H₂SO₄ bei einer Scangeschwindigkeit von 40 mV/s durchgeführt. Der Potential-Bereich lag dabei zwischen -0.3 und +1.2 V gemessen gegen die Standard-Kalomel Elektrode (SCE).
Vom 1. zum 100. Zyklus ist bei der iridiumbeschichteten Elektrode im Potential-Bereich zwischen ca. 0.6 und 1.2 V eine Zunahme des anodischen sowie des kathodischen Stroms zu erkennen. Dieser Prozess ist reversibel. Man spricht hierbei auch von einer Aktivierung der Ir-Schicht.

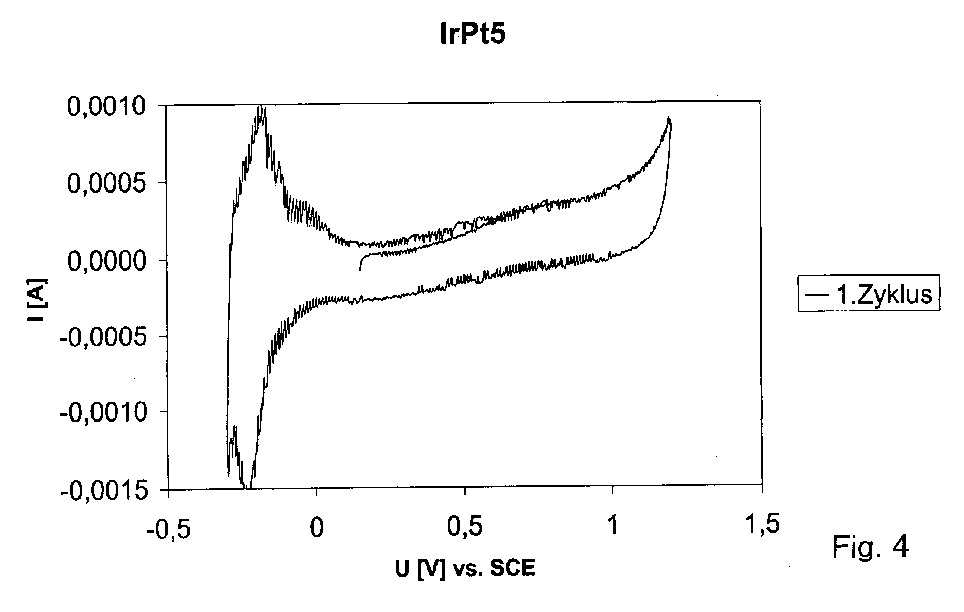

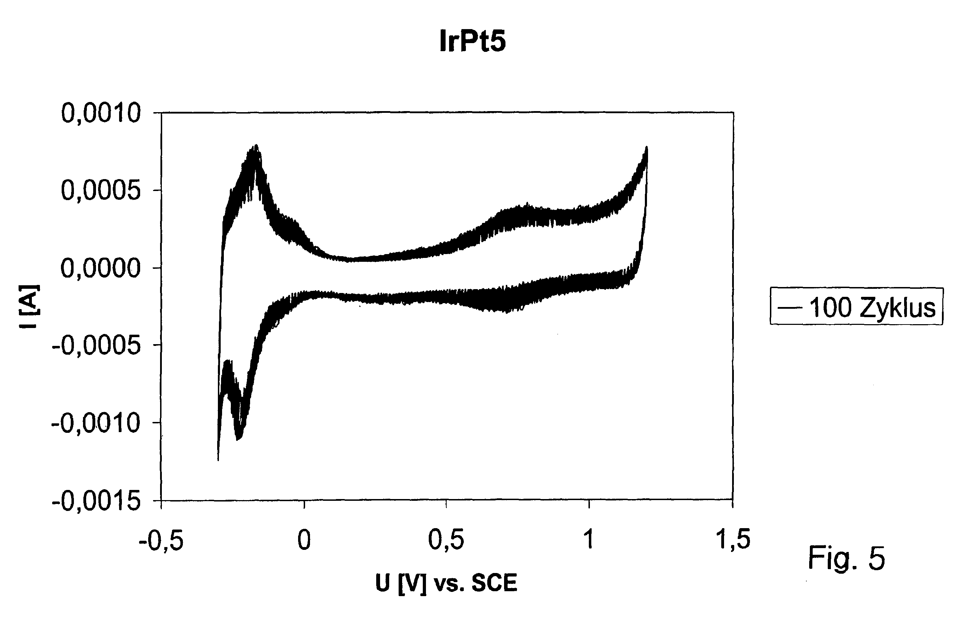

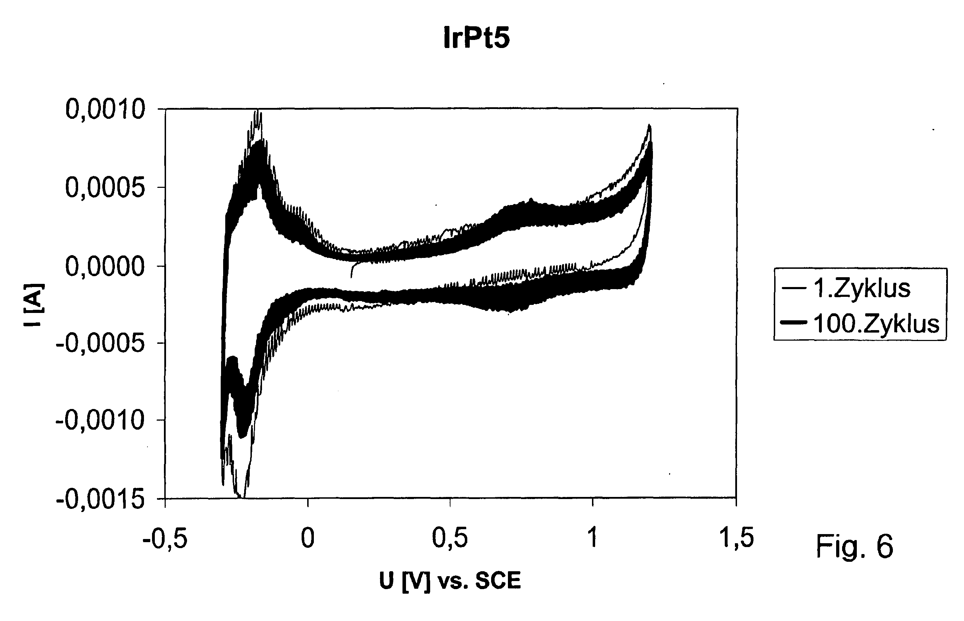

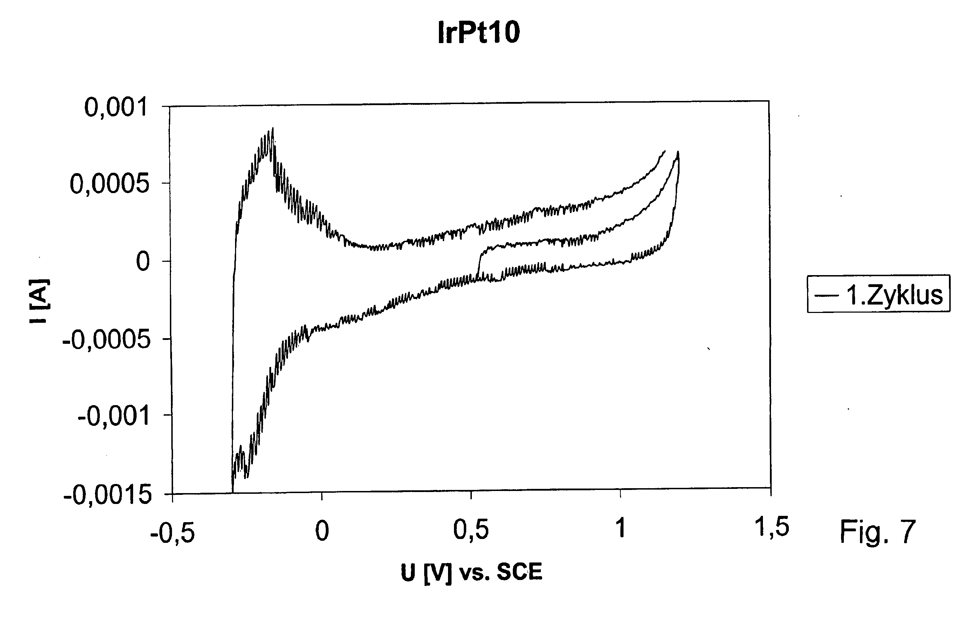

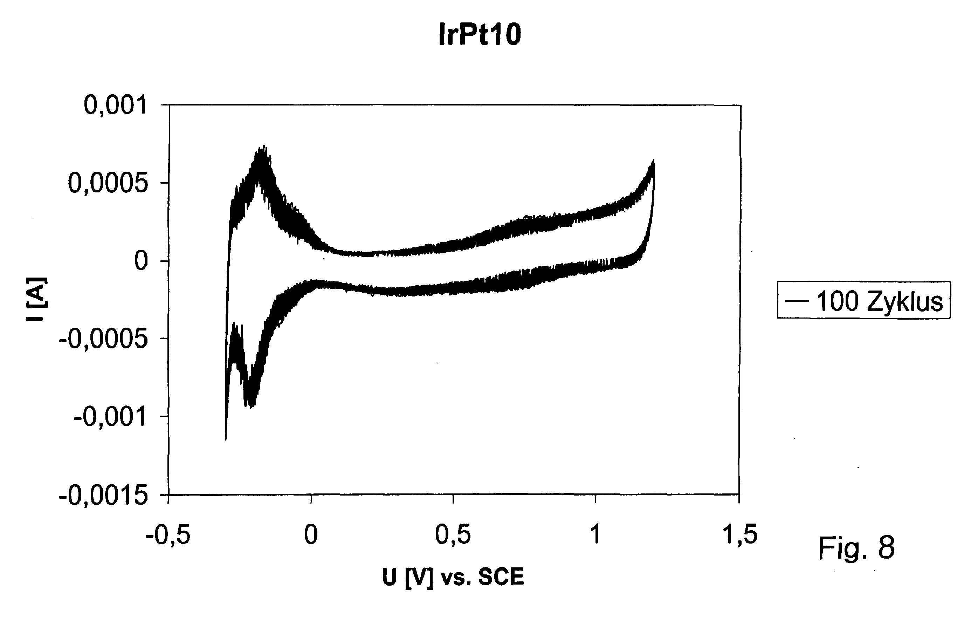

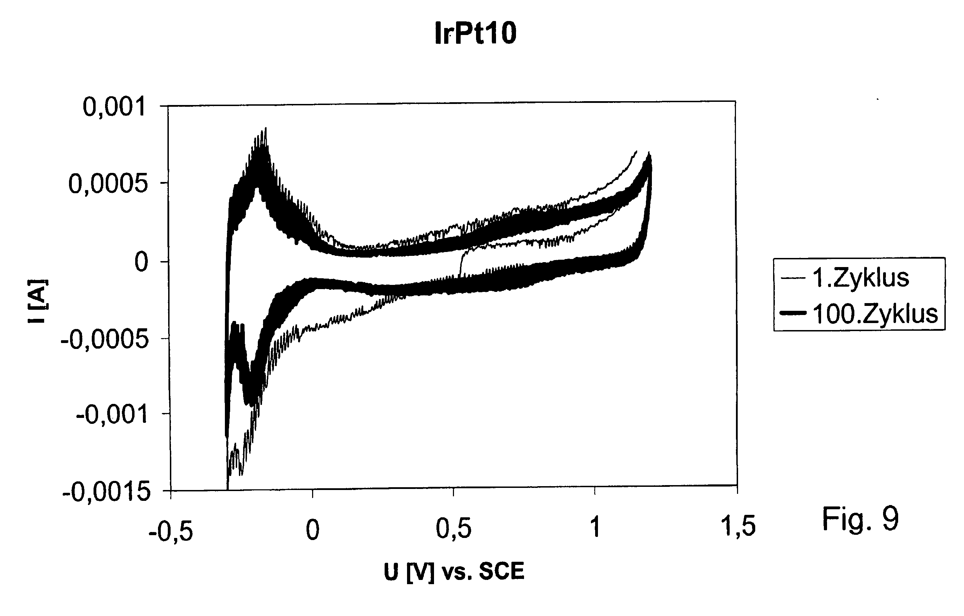

Die Figuren 4 bis 9 verdeutlichen den Einfluss einer Platin-Dotierung auf das elektrochemische Verhalten der Elektroden. Schon eine Platinkonzentration von ungefähr 5 % bewahrt die Elektrode im wesentlichen vor einer Aktivierung. Die Strom-Spannungskurve bleibt über 100 Zyklen nahezu konstant. Es findet also im Gegensatz zu Elektroden mit reiner Iridiumbeschichtung keine Zunahme des Strom im anodischen Potential-Bereich über 100 Zyklen statt. Bei einer 10 %igen Platin-Dotierung findet sogar eine leichte Abnahme des anodischen Stroms bis zum 100. Zyklus statt. (Figuren 7 bis 9).

### Herstellungsbeispiel:

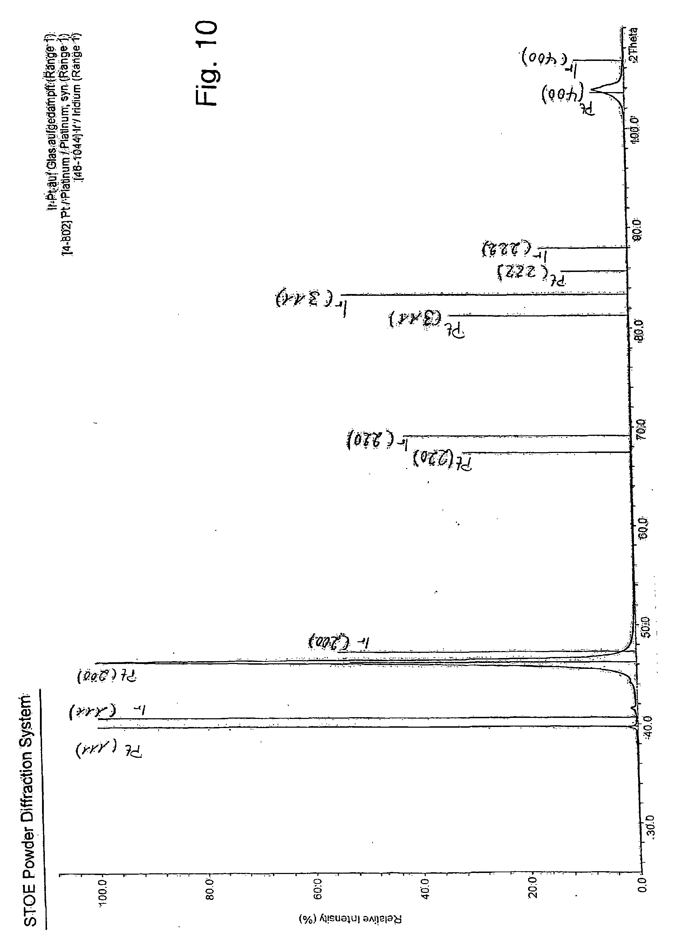

Eine Sputteranlage wird mit zwei Einrichtungen für zwei Targets ausgestattet.
Ein Platin- und ein Iridiumtarget werden in der Sputteranlage angeordnet. Hierauf wird in dieser Anlage ein Glaskörper beschichtet. Die Beschichtung wird mit einem Pulver-Diffraktometer-System analysiert (Figur 10). Der Peak bei (200) der Legierung ist gegenüber einem Platinpeak minimal in Richtung des Iridiumpeaks verschoben. Der Legierungspeak zeigt keine Aufspaltung, nicht einmal eine Schulter. Dies lässt darauf schließen, dass das Iridium vollständig im Platin gelöst ist, wobei die Platinstruktur im wesentlichen aufrechterhalten bleibt.

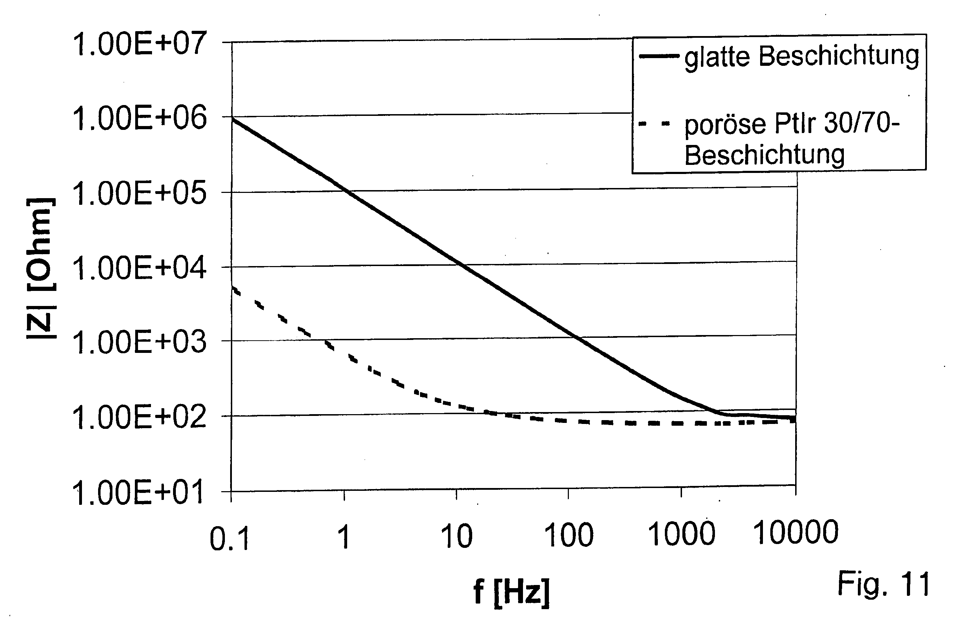

Erfindungsgemäß erfolgt ein poröser Legierungsauftrag der, wie in Figur 11 ersichtlich, in einem breiten Frequenzbereich, eine drastisch reduzierte Impedanz aufweist. Insbesondere ist ein Anstieg der Impedanz erst im Frequenzbereich zwischen 0,1 und 10 Hz merklich, während ein Anstieg bei glatter Beschichtung durch galvanischen Auftrag bereits über 1000 Hz erfolgt. Dagegen bleibt die Impedanz der porösen Platinlegierung bis hinunter zu 100 Hz fast konstant und bis ungefähr hinunter zu 10 Hzz nur geringfügig verändert. Mit der erfindungsgemäß durch simultane Besputterung erhaltenen porösen Legierungsschicht ist bei einer Frequenz im Bereich zwischen 0,1 und 1000 Hz eine drastische Verbesserung der Stimmulationseigenschaften wie die Pulsübertragung am Elektode-Gewebe-Übergang gewährleistet. Mit einer durch galvanische Beschichtung erzeugten glatten Schicht wurden bei 100 Hz 1,8 µF gemessen, bei 1 Hz 2 µF und bei 10 mHz 5 µF, wogegen mit der erfindungsgemäßen porösen Schicht bei 100 Hz 120 µF gemessen wurden, bei 1 Hz 279 µF und bei 10 mH 409 µF. Die hierzu verwendete erfindungsgemäße poröse Schicht ist eine Iridium-Platin 70:30 Legierung. Im Legierungsbereich 70:30 bis 30:70 sind keine Unterschiede feststellbar.

### Anwendunpsbeispiel

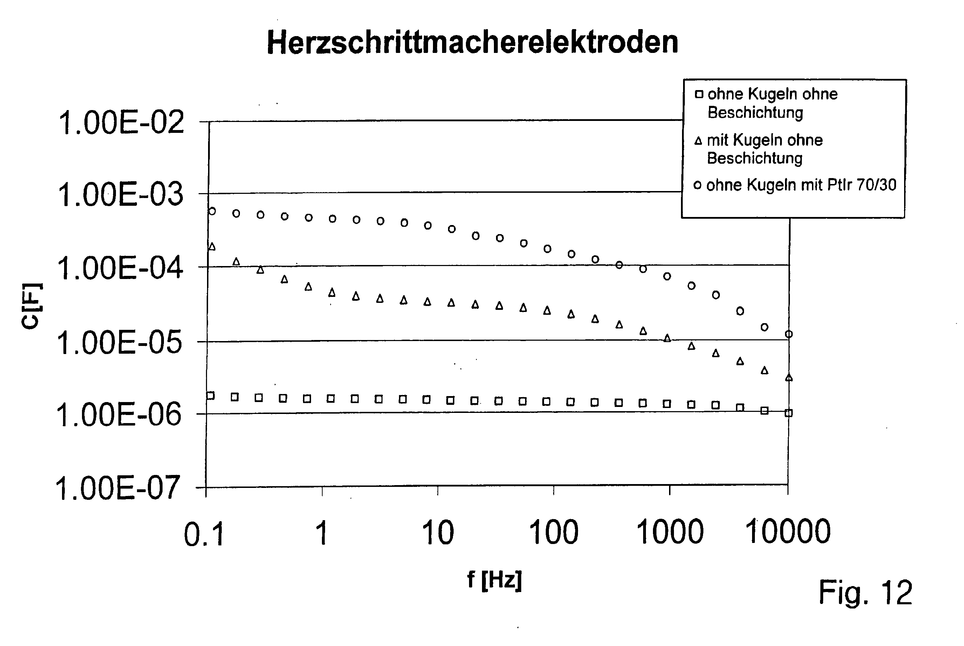

In Figur 12 wird die Kapazitätssteigerung gegenüber einer unbeschichteten Elektrode aufgezeigt. Dabei ist bereits mit einem Spheroidauftrag eine Kapazitätssteigerung realisierbar. Hierüber hinaus lässt sich eine weitere Steigerung der Kapazität durch eine Beschichtung mit einer Platin-Iridium-Legierung erzielen, die mittels zweier Sputtertargets, einem aus Iridium und dem anderen aus Platin in einem PVD-Verfahren aufgetragen wurde. Die unbeschichtete Elektrode hatte bei 100 Hz eine Kapazität von 1,4 µF und bei 1 Hz 1,6 µF. Mit einem Auftrag von 10 mg an Platin-Iridium-Spheroiden (Kugeln) wurde die Kapazität bei 100 Hz auf 22,9 µF gesteigert und bei 1 Hz auf 44,6 µF. Dem gegenüber wurde mit geringerem Edelmetalleinsatz eine weitere Steigerung der Kapazität geschaffen, indem 0,5 mg Beschichtung von einem Iridiumtarget und einem Platintarget bei simultaner PVD-Beschichtung auf dem Substrat aufgetragen wurden. Dabei wurden bei 100 Hz 143 µF und bei 1 Hz 440 µF erzielt. Das Verhältnis von Kapazität pro eingesetztem Edelmetall ist daher bei 100 Hz erfindungsgemäß über 60 mal größer als bei einer Beschichtung mit Kugeln und bei 1 Hz sogar fast 200 mal größer. Damit ist gezeigt, dass der erfindungsgemäße Auftrag bedeutend effizienter ist als die Beschichtung mit Spheroiden.

## Patentansprüche

1. Elektrode für medizinische Anwendungen, wobei die Elektrode zumindest teilweise mit einer porösen Platin-Iridium (Ptir) Schicht beschichtet ist, **dadurch gekennzeichnet, dass** die Beschichtung mehr als 20 Ges.-% Iridium (Ir) und mindestens 100 ppm Platin (Pt) enthält und der beschichtete Bereich in physiologischer Kochsalzlösung bei 37°C und einer Messfrequenz von 100 mHz eine elektrochemische Kapazität grösser als 5 mF/cm² aufweist.

2. Elektrode nach Anspruch 1, **dadurch gekennzeichnet, dass** die Elektrode einen Grundkörper aus Titan (Ti), Tantal (Ta), Platin (Pt), Platin-Iridium (Ptlr) oder einer Kobalt-Chrom (CoCr)-Legierung aufweist.

3. Elektrode nach Anspruch 1 oder 2, **dadurch gekennzeichnet, dass** die Beschichtung weniger als 1 mg Edelmetall aufweist.

4. Elektrode nach Anspruch 3, **dadurch gekennzeichnet, dass** die Platin-Iridium (Ptir)-Beschichtung mehr als 40 Ges.-% Iridium (Ir) aufweist.

5. Elektrode nach Anspruch 4, **dadurch gekennzeichnet, dass** die Platin-Iidium (Ptlr)-Beschichtung mehr als 60 % Iridium (Ir) aufweist.

6. Elektrode nach Anspruch 5, **dadurch gekennzeichnet, dass** die Platin-Iridium (Ptlr)-Beschichtung mehr als 80 % Iridium (Ir) aufweist.

7. Elektrode nach einem der Ansprüche 1 bis 6, **dadurch gekennzeichnet, dass** in der Platin-Iridium (Ptlr)-Beschichtung das Platin vollständig im Iridium gelöst ist.

8. Elektrode insbesondere nach einem der Ansprüche 1 bis 7, **dadurch gekennzeichnet, dass** die Elektrode eine poröse Platin-Iridium (Ptlr)-Beschichtung mit einem Gradienten in der Stöchiometrie aufweist.

9. Elektrode nach Anspruch 8, **dadurch gekennzeichnet, dass** die Elektrode eine poröse Platin-Iridium (Ptlr)-Beschichturig aufweist, In der mindestens ein Legierungsbestandteil über die Schichtdicke um mindestens 10 % variiert.

10. Sputterverfahren zur Herstellung einer Elektrode nach einem der Ansprüche 1 bis 9 für medizinische Anwendungen, **dadurch gekennzeichnet, dass** auf die Elektrode simultan mit mindestens einem Iridiumtarget und mindestens einem Platintarget eine poröse Platin-Iridium-Schicht aufgetragen wird.

11. Sputterverfahren nach Anspruch 10, **dadurch gekennzeichnet, dass** die Stöchiometrie der Platin-Iridium-Beschichtung während des Beschichtungsprozesses variiert wird.

## Claims

1. Electrode for medical applications, whereby the electrode is coated, at least partly, by a porous platinum-iridium (Ptlr) layer, **characterized in that**
the coating contains more than 20 wt.-% iridium (Ir) and at least 100 ppm platinum (Pt) and the coated region has an electrochemical capacitance in excess of 5 mF/cm² in physiological saline at 37°C and at a measuring frequency of 100 mHz.

2. Electrode according to claim 1, **characterized in that** the electrode has a base body made of titanium (Ti), tantalum (Ta), platinum (Pt), platinum-iridium (Ptlr) or a cobalt-chromium (CoCr) alloy.

3. Electrode according to claim 1 or 2, **characterized in that** the coating comprises less than 1 mg precious metal.

4. Electrode according to claim 3, **characterized in that** the platinum-iridium (Ptlr) coating comprises in excess of 40 wt.-% iridium (Ir).

5. Electrode according to claim 4, **characterized in that** the platinum-iridium (Ptlr) coating comprises in excess of 60 wt.-% iridium (Ir).

6. Electrode according to claim 5, **characterized in that** the platinum-iridium (Ptlr) coating comprises in excess of 80 wt.-% iridium (Ir).

7. Electrode according to any one of the claims 1 to 6, **characterized in that** the platinum is completely dissolved in the iridium in the platinum-iridium (Ptlr) coating.

8. Electrode in particular according to any one of the claims 1 to 7, **characterized in that** the electrode comprises a porous platinum-iridium (Ptlr) coating with a gradient in its stoichiometry.

9. Electrode according to claim 8, **characterized in that** the electrode comprises a porous platinum-iridium (Ptlr) coating, in which at least one alloy component varies by at least 10 % over the thickness of the layer.

10. Sputtering method for the production of an electrode according to any one of the claims 1 to 9 for medical applications, **characterized in that** a porous platinum-iridium layer is applied to the electrode using at least one iridium target and at least one platinum target simultaneously.

11. Sputtering method according to claim 10, **characterized in that** the stoichiometry of the platinum-iridium coating is varied during the coating process.

## Revendications

1. Electrode pour applications médicales, dans laquelle l'électrode est au moins partiellement revêtue d'une couche de platine-iridium (Ptlr) poreuse, **caractérisée en ce que** le revêtement contient plus de 20 % en poids d'iridium (Ir) et au moins 100 ppm de platine (Pt) et la zone revêtue présente, en solution physiologique de sel de cuisine à 37°C et à une fréquence de mesure de 100 mHz, une capacité électrochimique supérieure à 5 mF/cm².

2. Electrode selon la revendication 1, **caractérisée en ce que** l'électrode présente un corps de base en titane (Ti), tantale (Ta), platine (Pt), platine-iridium (Ptlr) ou un alliage de cobalt-chrome (CoCr).

3. Electrode selon la revendication 1 ou 2, **caractérisée en ce que** le revêtement présente moins de 1 mg de métal précieux.

4. Electrode selon la revendication 3, **caractérisée en ce que** le revêtement de platine-iridium (Ptlr) présente plus de 40 % en poids d'iridium (Ir).

5. Electrode selon la revendication 4, **caractérisée en ce que** le revêtement de platine-iridium (Ptlr) présente plus de 60 % en poids d'iridium (Ir).

6. Electrode selon la revendication 5, **caractérisée en ce que** le revêtement de platine-iridium (Ptlr) présente plus de 80 % en poids d'iridium (Ir).

7. Electrode selon l'une quelconque des revendications 1 à 6, **caractérisée en ce que** le platine est entièrement dissous dans l'iridium dans le revêtement de platine-iridium (Ptlr).

8. Electrode notamment selon l'une quelconque des revendications 1 à 7, **caractérisée en ce que** l'électrode présente un revêtement de platine-iridium (Ptlr) poreux avec un gradient dans la stoechiométrie.

9. Electrode selon la revendication 8, **caractérisée en ce que** l'électrode présente un revêtement de platine-iridium (Ptlr) poreux, dans lequel au moins un constituant de l'alliage varie d'au moins 10 % en ce qui concerne son épaisseur de couche.

10. Procédé de pulvérisation pour la fabrication d'une électrode selon l'une quelconque des revendications 1 à 9 pour applications médicales, **caractérisé en ce qu'**une couche de platine-iridium poreuse est appliquée sur l'électrode en même temps qu'au moins une cible d'iridium et au moins une cible de platine.

11. Procédé de pulvérisation selon la revendication 10, **caractérisé en ce que** la stoechiométrie du revêtement de platine-iridium varie pendant le processus de revêtement.
